# EUROPEAN PATENT SPECIFICATION

(11) **EP 0 732 117 B1**
(45) Date of publication and mention of the grant of the patent: **11.08.1999**
(21) Application number: 96301584.7
(22) Date of filing: 07.03.1996
(51) Int. Cl.: A61M 25/00

(54) **A catheter structure for use in medical treatment**
Katheterstruktur für medizinische Behandlung
Structure de cathéther pour traitements médicaux

(30) Priority: 17.03.1995 JP 5894095
(43) Date of publication of application: 18.09.1996
(73) Proprietor: Asahi Intecc Co., Ltd., Seto-shi, Aichi-ken (JP)
(72) Inventor: Miyata, Kenji, Seto-shi, Aichi-ken (JP); Kato, Tadakazu, Seto-shi, Aichi-ken (JP); Mizukami, Isamu, Seto-shi, Aichi-ken (JP)
(74) Representative: Senior, Alan Murray

(56) References cited:
- EP-A- 0 358 117
- WO-A-96/00101
- US-A- 5 019 057
- US-A- 5 057 092

## Description

The invention relates to a catheter structure usually used for medical treatment to improve its manipulability.

In a catheter structure which has an synthetic inner tube, a metallic mesh knit provided to surround an outer surface of the inner tube and an outer tube provided to cover an outer surface of the metallic mesh knit, the metallic mesh knit is made of flat thin wires of the same mechanical strength substantially knitted with equal pitch and angle.

In this type of the catheter, it is difficult to concurrently overcome conflicting requirements between its structural property and functional property. When the thickness of the metallic mesh knit is reduced, its kink resistance property and good torque transmission is sacrificed although it better follows up a blood vessel due to an increased flexibility. When the thickness of the metallic mesh knit is increased, its follow-up property and inner diameter are reduced with less flexibility although the kink resistance property and good torque transmission are improved.

With the reduced diameter of the metallic mesh knit, there arises a problem of increasing vessel pressure when supplying contrast medium with the blood vessel. When a guide catheter is used, there arises a problem of impeding passage of a balloon catheter and a stent.

US 5,019,057 discloses a catheter structure in accordance with the preamble of claim 1.

Therefore, it is an aim of the invention to provide a catheter structure which is capable of improving a kink resistant and good torque transmission property while maintaining a good follow-up property without reducing its inner diameter.

Compared to US 5,019,057 the present invention is characterised by both the fibre or fibres of the one spiral line and the fibre or fibres of the other spiral line being rectangular in section, wherein the width of the fibre or fibres of the one spiral line being in the range of from 2.5 - 25 times that of the fibre or fibres of the other spiral line and the thickness of the fibre or fibres of the one spiral line being 1.0 - 10 times that of the fibre or fibres of the other spiral line;
the fibre or fibres of the one spiral line being greater than the fibre or fibres of the other spiral line in hardness; and
the fibre or fibres of the one spiral line being greater than the fibre or fibres of the other spiral line in terms of geometrical moment of inertia.

Further optional features of the invention are set out in the dependent claims appended hereto.

These, together with other aims, features and advantages which will be further described hereinafter with reference to exemplary embodiments and the accompanying drawings, in which:
Fig. 1a is a perspective view of a catheter structure according to an embodiment of the invention;
Fig. 1b is an enlarged perspective view of a main part of the catheter structure taken along the line A-A of Fig. 1a but partly removed; and
Figs 2a-2d are sequential views of the main part of the catheter structure showing how the catheter is assembled.

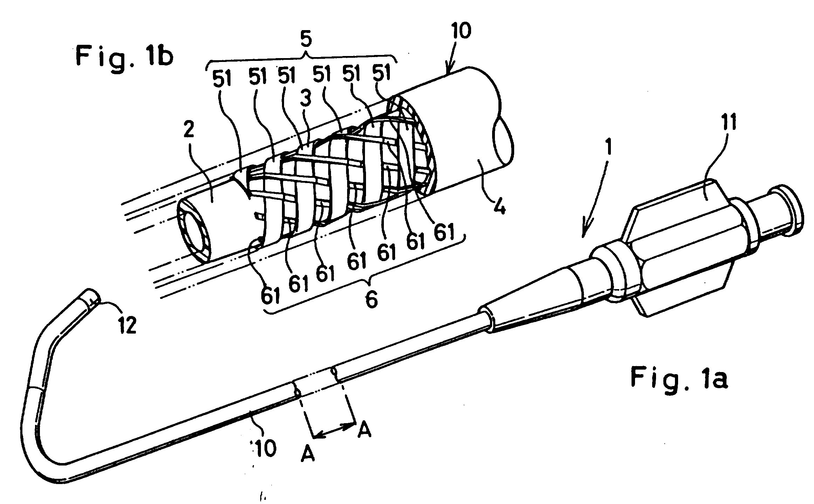

Referring to Fig. 1a, a catheter 1 has a catheter body 10 and a grip 11 provided at a rear portion of the catheter body 10. To a front end surface of the catheter body 10 which is made of a flexible tube, a soft tip 12 is connected. As shown in an enlarged view in Fig. 1b, the catheter body 10 has an inner tube 2 made of a synthetic resin. An outer surface of the inner tube is surrounded by a metallic mesh knit 3 which is coated by an outer tube 4 made of a synthetic resin. The metallic mesh knit 3 comprises a series of counterclockwise spiral lines and a series of clockwise spiral lines 6.

The spiral line 5 is adapted to be mechanically stronger than another spiral line 6 in the following manner.

The spiral line 5 includes eight thin fibres 51, each of which is flatly compressed into sash-like structure which measures 0.26 mm in width and 0.06 mm in thickness. Each of the thin fibers 51 is originally made of a stainless steel wire which measures 0.12 mm in diameter. In this instance, the stainless steel wire of the thin fiber 51 is usable so long as its diameter is below 0.2 mm with its Vicker's hardness (Hv) ranging from 180 to 750. It is preferable that the compressed thin fiber 51 measure 0.05 mm - 0.50 mm in width and 0.01 mm - 0.10 mm in thickness. The number of the thin fibers 51 can be altered appropriately within the range of 1 - 24.

On the other hand, another spiral line 6 includes eight thin fibers 61, each of which is flatly compressed into sash-like structure which measures 0.13 mm in width and 0.03 mm in thickness. Each of the thin fibres 61 is originally made of an annealed stainless steel wire which measures 0.07 mm in diameter. In this instance, the stainless steel wire of the thin fiber 61 is usable so long as its diameter ranges from 0.04 mm to 0.12 mm with its Vicker's hardness (Hv) ranging from 50 to 210. It is preferable that the thin fiber 61 measures 0.02 mm - 0.20 mm in width and 0.01 mm - 0.10 mm in thickness. The number of the thin fibers 51 can be altered appropriately within the range of 1 - 24.

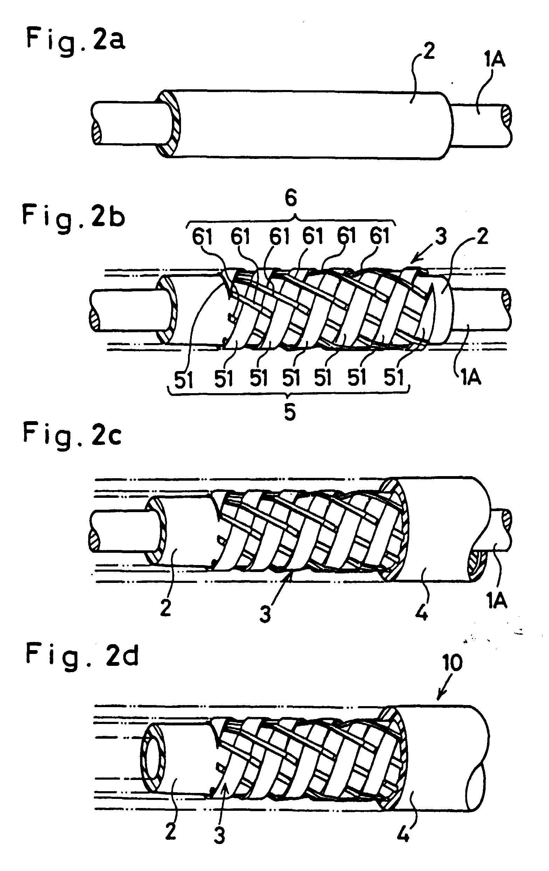

The method of manufacturing the catheter 1 is as follows:
(1) The inner tube 2 is made by extruding or blowing the synthetic resin over an outer surface of a metallic core 1A as shown in Fig. 2a.
(2) On the outer surface of the inner tube 2, the eight numbers of thin fibers 51 and the thin fibers 61 are knitted together to form the metallic mesh knit 3 as shown in Fig. 2b.
(3) After knitting the thin fibers 51, 61, the thin fiber 61 is yieldingly bent to form a hook portion against another thin fiber 51 at each of the intersection of the thin fibers 51, 61.
(4) The outer tube 4 is made by extruding or blowing the synthetic resin over the outer surface of the metallic mesh knit 3 as shown in Fig. 2c.
(5) The metallic core 1A is axially stretched to reduce its diametrical dimension, and the core 1A is pulled out of the inner tube 2 as shown in Fig. 2d.

With the thin fiber 61 yieldingly bent against another thin fiber 51, the thin fiber 61 entangles with another thin fiber 51 so as to increase the mechanical strength of the metallic mesh knit 3. This makes it possible for the catheter to favorably follow up the blood vessel while maintaining a good kink resistant and torque transmission property.

In the foregoing embodiment of the invention, the cross sectional area and the hardness are discretionally altered to provide the difference of the mechanical strength between the two types of the thin fiber 51, 61, the difference of the mechanical strength may be added as follows:
(a) Instead of the annealed stainless steel, the thin fiber 61 of the spiral line 6 may be made by polyamide, polypropylene or the like.
(b) In the method of manufacturing the catheter, a step of compressing the metallic mesh knit 3 is not always necessary. Without compressing the metallic mesh knit, the thin fiber 61 is yieldingly bent against the thin fiber 51 at their intersection, thus substantially improving the kink resistant and torque transmission property.

While the invention has been described with reference to the specific embodiments, it is understood that this description Is not to be construed in a limiting sense in as much as various modifications and additions to the specific embodiments may be made by skilled artisan without departing from the scope of the invention, as defined in the appended claims.

## Claims

1. A catheter structure comprising:
an inner tube (2);
an at least partially metallic mesh knit (3) surrounding an outer surface of the inner tube (2), and
an outer tube (4) covering an outer surface of the metallic mesh knit (3);
wherein the metallic mesh knit (5) is formed by two spiral lines (5, 6) one of which is greater than the other in mechanical strength, each of said spiral lines (5, 6) comprises a single thin fibre or a plurality of thin fibres, the fibre or fibres of the one spiral line (5) being greater in cross section than the fibre or fibres of the other spiral line (6);
characterised by both the fibre or fibres of the one spiral line and the fibre or fibres (51) of the other spiral line being rectangular in section, wherein the width of the fibre or fibres of the one spiral line (5) being in the range of from 2.5 - 25 times that of the fibre or fibres (61) of the other spiral line (6) and the thickness of the fibre or fibres (51) of the one spiral line (5) being 1.0 - 10 times that of the fibre or fibres (61) of the other spiral line (6);
the fibre or fibres of the one spiral line being greater than the fibre or fibres (61) of the other spiral line (6) in hardness; and
the fibre or fibres (51) of the one spiral line (5) being greater than the fibre or fibres (61) of the other spiral line (6) in terms of geometrical moment of inertia.

2. A catheter structure according to claim 1, wherein the one spiral line (5, 6) is made of stainless steel, and the other spiral line is made of synthetic resin.

3. A catheter structure according to claim 1 or 2, wherein each of the two spiral lines (5, 6) is comprised of a plurality of thin fibres, where a cross section of the fibres of one spiral line is substantially the same as that of the fibres of the other spiral line, and the number of the plurality of thin fibres of the one spiral line is greater than that of the other spiral line.

4. A catheter structure according to claim 1, wherein the number of the plurality of thin fibres of the one spiral line is in the range of from 1 to 24.

5. A catheter structure according to claim 1, the fibre or fibres of the one spiral line have dimensions in the range of 0.05 to 0.50 mm in width and 0.01 to 0.10 mm in thickness and the fibre or fibres of the other spiral line have dimensions in the range of 0.02 to 0.20 mm in width and 0.01 to 0.10 mm in thickness.

6. A catheter structure according to claim 1, wherein each of the two spiral lines is made of stainless steel.

## Patentansprüche

1. Katheterstruktur, umfassend:
einen Innenschlauch (2);
ein zumindest teilweise metallisches Maschengeflecht (3), das eine Außenfläche des Innenschlauchs (2) umgibt, und
einen Außenschlauch (4), der eine Außenfläche des metallischen Maschengeflechts (3) abdeckt;
wobei das metallische Maschengeflecht (5) aus zwei Spiralsträngen (5, 6) gebildet wird, von denen einer eine größere mechanische Festigkeit als der andere besitzt, jede der Spiralstränge (5, 6) eine einzelne dünne Faser oder mehrere dünne Fasern enthält, wobei die Faser oder Fasern des einen Spiralstrangs (5) einen größeren Querschnitt besitzt bzw. besitzen als die Faser oder die Fasern des anderen Spiralstrangs (6);
**dadurch gekennzeichnet**, daß sowohl die Faser oder die Fasern des einen Spiralstrangs als auch die Faser oder die Fasern (51) des anderen Spiralstrangs einen rechteckigen Querschnitt besitzen, bei dem die Breite der Faser oder der Fasern des einen Spiralstrangs (5) im Bereich des 2,5- bis 25-fachen derjenigen der Faser oder der Fasern (61) des anderen Spiralstrangs (6) liegt und die Dicke der Faser oder der Fasern (51) des einen Spiralstrangs (5) dem 1,0- bis 10-fachen derjenigen der Faser oder der Fasern (61) des anderen Spiralstrangs (6) entspricht;
die Faser oder die Fasern des einen Spiralstrangs eine größere Härte besitzt bzw. besitzen als die Faser oder die Fasern (61) des anderen Spiralstrangs (6); und
die Faser oder die Fasern (51) des einen Spiralstrangs (5) ein größeres geometrisches Trägheitsmoment besitzt bzw. besitzen als die Faser oder die Fasern (61) des anderen Spiralstrangs (6).

2. Katheterstruktur nach Anspruch 1, bei der der eine Spiralstrang (5, 6) aus rostfreiem Stahl besteht und der andere Spiralstrang aus Kunstharz besteht.

3. Katheterstruktur nach Anspruch 1 oder 2, bei der jeder der beiden Spiralstränge (5, 6) aus einer Mehrzahl dünner Fasern gebildet ist, wobei ein Querschnitt der Fasern eines Spiralstrangs im wesentlichen der gleiche ist wie derjenige der Fasern des anderen Spiralstrangs, und die Anzahl der mehreren dünnen Fasern des einen Spiralstrangs größer ist als diejenige des anderen Spiralstrangs.

4. Katheterstruktur nach Anspruch 1, bei der die Anzahl der mehreren dünnen Fasern des einen Spiralstrangs im Bereich von 1 bis 24 liegt.

5. Katheterstruktur nach Anspruch 1, bei der die Faser oder die Fasern des einen Spiralstrangs Abmessungen im Bereich von 0,05 bis 0,50 mm in der Breite und 0,01 bis 0,10 mm in der Dicke besitzen, und die Faser oder die Fasern des anderen Spiralstrangs Abmessungen im Bereich von 0,02 bis 0,20 mm in der Breite und 0,01 bis 0,10 mm in der Dicke besitzen.

6. Katheterstruktur nach Anspruch 1, bei der jeder der beiden Spiralstränge aus rostfreiem Stahl besteht.

## Revendications

1. Structure de cathéter comprenant:
un tube intérieur (2);
un tissu à mailles (3) au moins partiellement métallique entourant une surface extérieure du tube intérieur (2), et
un tube extérieur (4) couvrant une surface extérieure du tissu à mailles métallique (3);
dans laquelle le tissu à mailles métallique (5) est formé par deux lignes en spirale (5, 6) dont l'une a une plus grande résistance mécanique que l'autre, chacune desdites lignes en spirale (5, 6) comporte une seule fibre mince ou une pluralité de fibres minces, la fibre ou les fibres de la première ligne en spirale (5) ayant une section transversale plus grande que celle de la fibre ou des libres de l'autre ligne en spirale (6);
caractérisée en ce que la fibre ou les fibres de la première ligne en spirale et la fibre ou les fibres (51) de l'autre ligne en spirale ont toutes une section rectangulaire, la largeur de la fibre ou des fibres de la première ligne en spirale (5) étant comprise entre 2,5 et 25 fois celle de la fibre ou des fibres (61) de l'autre ligne en spirale (6) et l'épaisseur de la fibre ou des fibres (51) de la première ligne en spirale (5) étant de 1,0 A 10 fois celle de la fibre ou des fibres (61) de l'autre ligne en spirale (6);
la fibre ou les fibres de la première ligne en spirale ayant une plus grande dureté que la fibre ou les fibres (61) de l'autre ligne en spirale (6); et
la fibre ou les fibres (51) de la première ligne en spirale (5) ayant un moment géométrique d'inertie supérieur à celui de la fibre ou des fibres (61) de l'autre ligne en spirale (6).

2. Structure de cathéter selon la revendication 1, dans laquelle la première ligne en spirale (5, 6) est en acier inoxydable, et l'autre ligne en spirale est en résine synthétique.

3. Structure de cathéter selon la revendication 1 ou 2, dans laquelle chacune des deux lignes en spirale (5, 6) est constituée par une pluralité de fibres minces, une section transversale des fibres d'une première ligne en spirale étant sensiblement la même que celle des fibres de l'autre ligne en spirale, et le nombre de la pluralité de fibres minces de la première ligne en spirale étant supérieur à celui de l'autre ligne en spirale.

4. Structure de cathéter selon la revendication 1, dans laquelle le nombre de fibres de la pluralité de fibres minces de la première ligne en spirale est compris entre 1 et 24.

5. Structure de cathéter selon la revendication 1, la fibre ou les fibres de la première ligne en spirale ayant des dimensions comprises entre 0,05 et 0,50 mm en largeur et 0,01 et 0,10 mm en épaisseur et la fibre ou les fibres de l'autre ligne en spirale ayant des dimensions comprises entre 0,02 et 0,20 mm en largeur et 0,01 et 0,10 mm en épaisseur.

6. Structure de cathéter selon la revendication 1, dans laquelle chacune des deux lignes en spirale est en acier inoxydable.
